(19) Europäisches Patentamt
European Patent Office
Office européen des brevets

(11) **EP 3 451 991 B1**

(12) **EUROPEAN PATENT SPECIFICATION**

(45) Date of publication and mention
of the grant of the patent:
**02.09.2020 Bulletin 2020/36**

(21) Application number: **17723894.6**

(22) Date of filing: **05.05.2017**

(51) Int Cl.:
**A61F 13/49** *(2006.01)*

(86) International application number:
**PCT/US2017/031298**

(87) International publication number:
**WO 2017/192992 (09.11.2017 Gazette 2017/45)**

(54) **ABSORBENT ARTICLES COMPRISING ELASTICS IN THE CROTCH REGION**

SAUGFÄHIGE ARTIKEL MIT GUMMIBÄNDERN IM SCHRITTBEREICH

ARTICLES ABSORBANTS COMPRENANT DES ÉLASTIQUES DANS LA RÉGION D'ENTREJAMBE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.05.2016 US 201662332496 P**

(43) Date of publication of application:
**13.03.2019 Bulletin 2019/11**

(73) Proprietor: **The Procter & Gamble Company
Cincinnati, OH 45202 (US)**

(72) Inventors:
• **DESAI, Fred Naval
Cincinnati
Ohio 45202 (US)**
• **MINOGUCHI, Ryo
Cincinnati
Ohio 45202 (US)**

(74) Representative: **P&G Patent Germany
Procter & Gamble Service GmbH
Sulzbacher Straße 40
65824 Schwalbach am Taunus (DE)**

(56) References cited:
**EP-A1- 2 695 590     US-A1- 2008 051 755**

**Description**

FIELD OF THE INVENTION

The present invention relates to disposable pull-on garments comprising elastics in the crotch region.

BACKGROUND OF THE INVENTION

The present specification discloses disposable absorbent articles comprising central panels (also referred to as central chassis or center chassis or main body) comprising absorbent cores. The absorbent articles may also comprise graphics. The disclosed designs of articles herein may be worn by all ages, including infants, toddlers, children, as well as adults.

It may be desirable to run elastics continuously across the core and/or central panel to decrease the appearance of the core and central panel area, which may help the absorbent article to appear more underwear like. This may be especially important for adult wearers. Running elastics continuously across the core and/or central panel may create too much tension in force zones and/or elastic sections causing the crotch area and areas adjacent to the crotch area to pull in transversely (i.e., too narrow).

Due to this, absorbent articles are often designed with elastics that are cut so they do not run across or continuously across the core or the central panel. Elastics cut in this manner may provide more coverage over the buttocks versus a product with elastics running continuously across the core and/or central panel. Of course, adequate coverage is desired for fit. Beyond fit, for adult incontinence products, adequate coverage is desired to provide comfort and security. Too much tension of a long continuous elastic can cause the portion of the belt adjacent to the transverse axis to narrow and ride up higher on the wearer's back-side compromising fit and causing discomfort.

Instead of cutting elastics to prevent the negatives of continuous elastics across the core and/or central panel, it may be desirable to space out elastics (i.e., decrease the number of elastics per area) in the force zones and/or elastic sections adjacent to the transverse centerline of the article. Thus, the area at and just above the leg cutout can benefit from spaced out elastics to keep tension from being too concentrated, thus causing narrowing of the front and back panels adjacent to the crotch. This is especially problematic in the back panel where it compromises buttocks coverage. With spaced out elastics, the average tension of the elastic zone and/or section is decreased.

One way to decrease the number of elastics per unit area is to create larger spaces between evenly, more tightly spaced elastics. Many elastic profiles of the present disclosure decrease the number of elastics per unit area by gradually increasing the space between elastics approaching the transverse centerline.

Additionally, it may also be desirable to create a larger area void of elastics on each side of the transverse axis for a certain area, which may simplify the process of making a multi-stranded elastic absorbent article because it creates a larger area without elastics. Elastics in this area may also be seen as unnecessary as the product at the center area of the crotch region is forced inwards by the wearer's thighs. Further, elastics in this region could overly narrow the crotch region such that gasketing is compromised. This may be especially true for articles comprising leg cuffs because the cuff path may be distorted by the lateral forces over the crotch region. Thus, this disclosure suggests desirable areas above and below the transverse centerline that may be free from transversely disposed elastics.

The disclosure also recognizes that gradual spacing changes of the elastic profile may be more aesthetically appealing versus cutting the elastics or providing large gaps throughout a profile.

BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which like designations are used to designate substantially identical elements, and in which:

FIG. 1 is a perspective view of an exemplary disposable pull-on garment in a typical in-use configuration;
FIG. 2 is a perspective view of an exemplary disposable pull-on garment in a typical in-use configuration;
FIGS. 3A-D are plan views of pull-on garments in their flat uncontracted condition showing the wearer-facing surface;
FIG. 4A is a schematic cross section view of a first embodiment taken along line 4A-4A in Figure 3A of an exemplary disposable pull-on garment;
FIG. 4B is a schematic cross section view of a second embodiment taken along line 4B-4B in Figure 3B of an exemplary disposable pull-on garment;
FIG. 5A is a schematic cross section view taken along line 5A-5A in FIG. 3A of an example of a leg cuff suitable in one embodiment of the invention.

FIG. 5B is a schematic cross section view taken along line 5B-5B in FIG. 3B of an example of a leg cuff suitable in one embodiment of the invention.

Fig. 6 is a schematic cross section view of suitable package of absorbent articles of the present disclosure.

DETAILED DESCRIPTION OF THE INVENTION

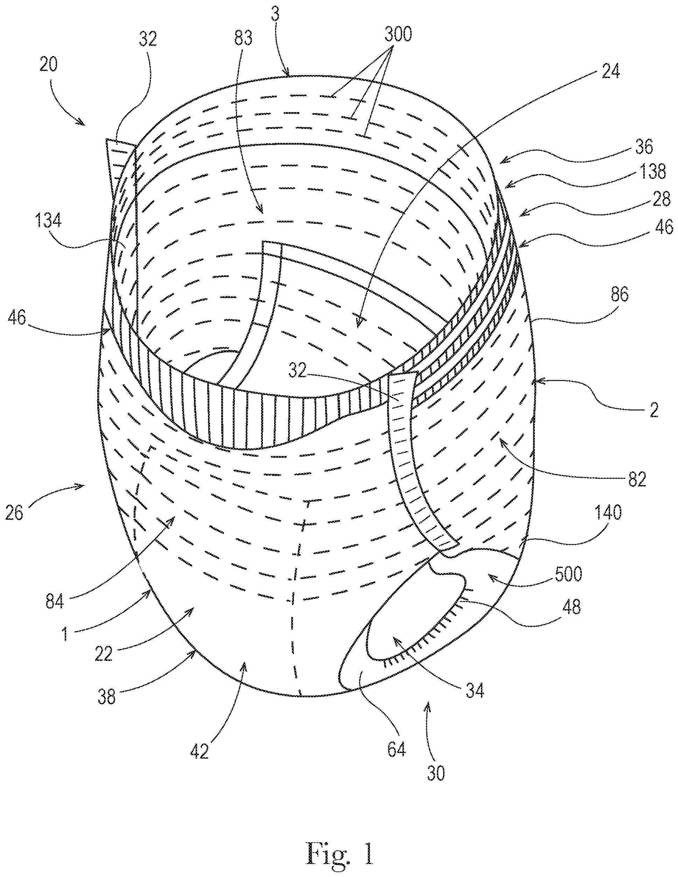

As used herein, the term "pull-on garment" refers to articles of wear which have a defined waist opening and a pair of leg openings and which are pulled onto the body of the wearer by inserting the legs into the leg openings and pulling the article up over the waist. The term "disposable" is used herein to describe garments which are not intended to be laundered or otherwise restored or reused as a garment (i.e., they are intended to be discarded after a single use and, preferably, to be recycled, composted or otherwise disposed of in an environmentally compatible manner). The pull-on garment is also preferably "absorbent" to absorb and contain the various exudates discharged from the body. A preferred embodiment of the absorbent article is the disposable absorbent pull-on garment, shown in Figure 1.

As used herein, the term "absorbent article" refers to pull-on garments generally worn by infants and other incontinent individuals to absorb and contain urine, feces and/or menses. It should be understood, however, that the term absorbent article is also applicable to other garments such as training pants, incontinent briefs, feminine hygiene garments or panties, and the like.

As used herein, the terms "elastic," "elastomer," and "elastomeric" refer to a material which generally is able to extend to a strain of at least 50% without breaking or rupturing, and is able to recover substantially to its original dimensions, accounting for set, after the deforming force has been removed.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

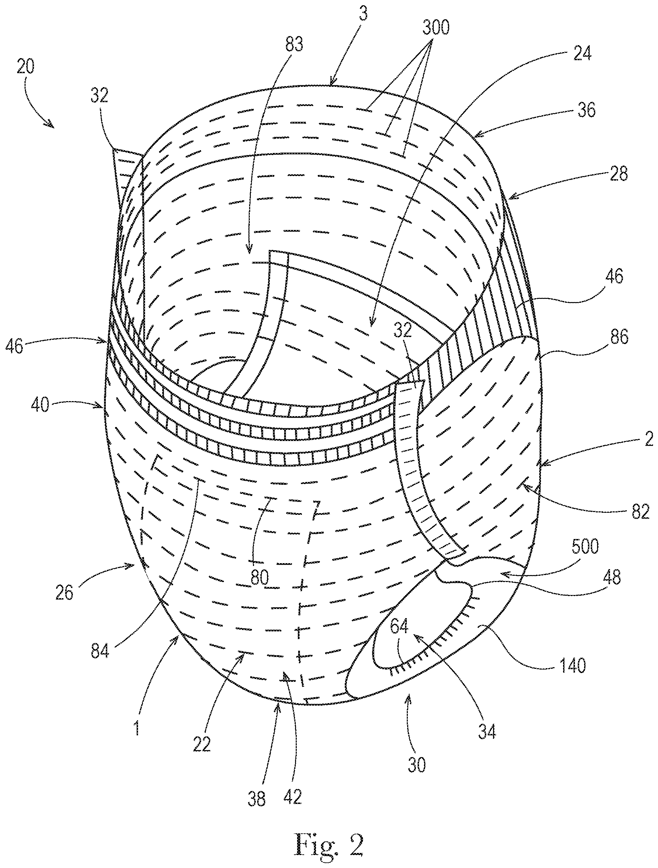

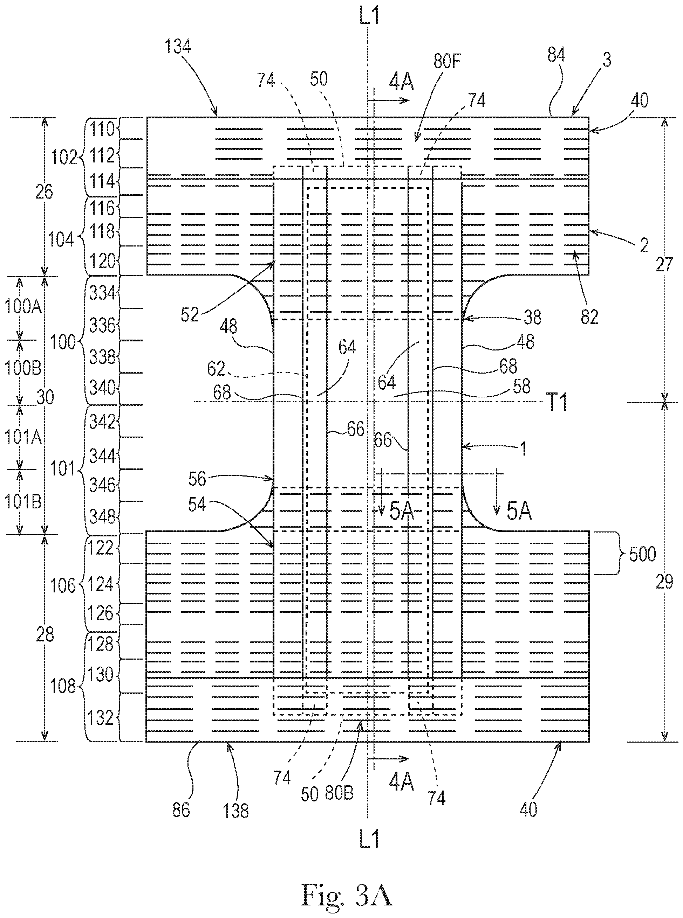

Figure 1 is a perspective view of the absorbent article 20. Figure 2 is a perspective view of the absorbent article 20. The absorbent article 20 has a longitudinal centerline Li and a transverse centerline T1 (refer to Figures 3A-D as well). The absorbent article 20 has an outer surface 22, an inner surface 24 opposed to the outer surface 22, a front region 26, a back region 28, a crotch region 30, and seams 32 which join the front region 26 and the back region 28 to form two leg openings 34 and a waist opening 36. Also referring to Figures 1-3, the absorbent article 20 comprises a main portion 1, a side portion 2, and a waist portion 3.

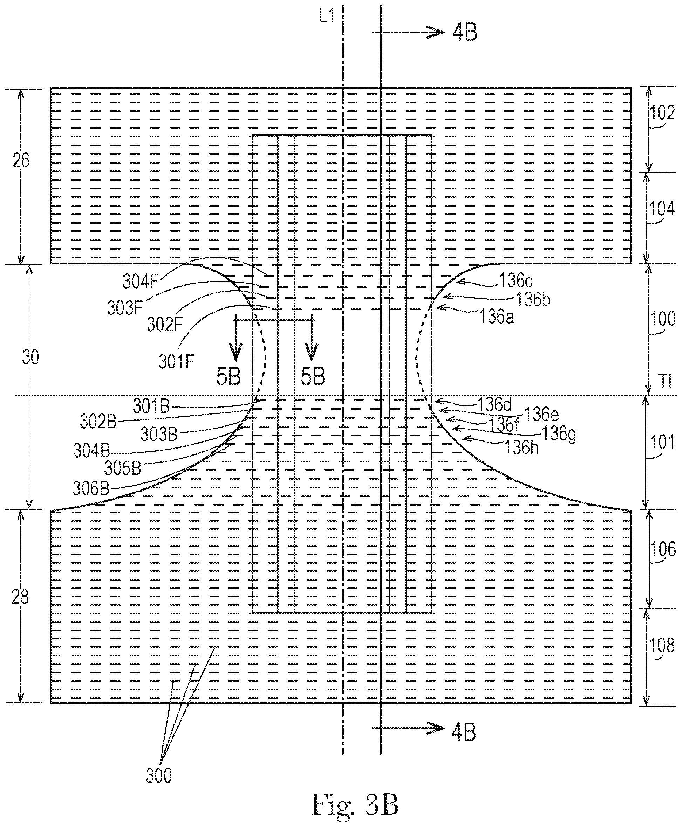

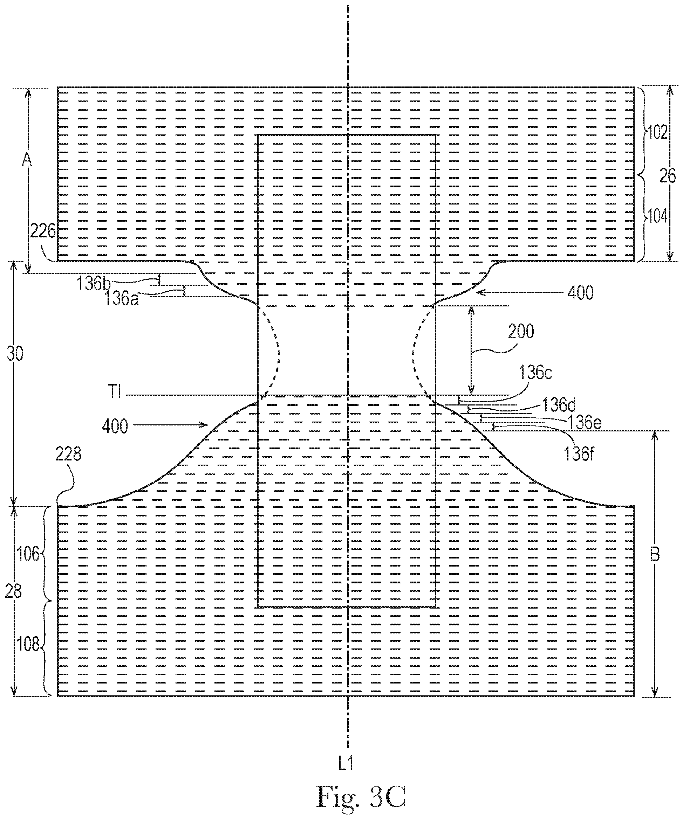

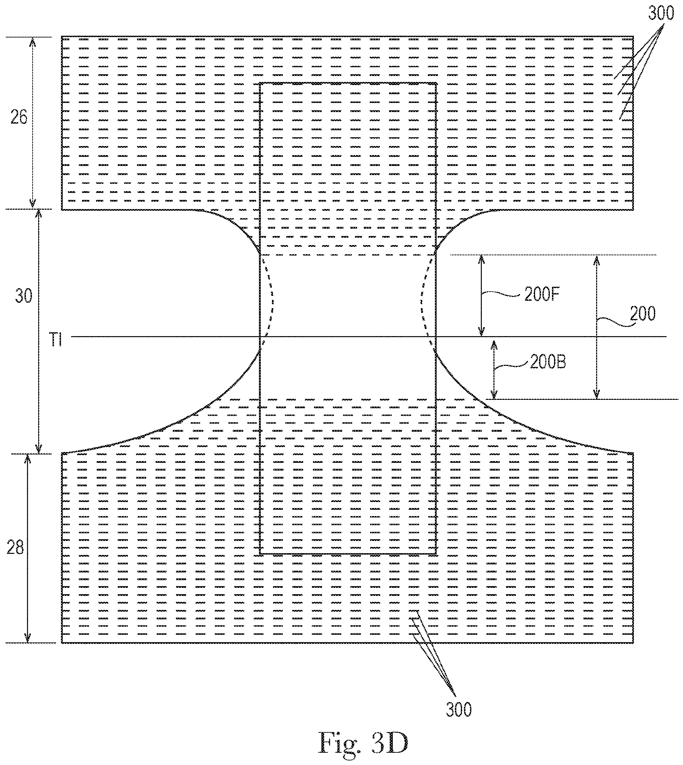

In the embodiment shown in Figures 1 and 3, the absorbent article 20 comprises an absorbent main body 38 (hereinafter may be referred to as "main body") to cover the crotch region of the wearer and a belt 40 extending transversely about the waist opening 36. The absorbent article 20 may also comprise an outer cover layer 42 to cover the main body 38. The belt 40 defines the waist opening 36. The belt 40, the main body 38 and/or the outer cover layer 42 jointly define the leg opening 34. The absorbent article 20 may have a graphic 46 disposed in the front region 26 and/or the back region 28. Graphics 46 may be printed on the outermost nonwovens (e.g. the outer belt layer 82, or the outer cover layer 42), either on the garment facing side or the body facing side of these nonwovens. Graphics may also be printed on the garment facing side of the inner belt layer 83. Graphics may also be printed on a garment-facing side of the backsheet film. Suitable graphics and graphic configurations for the absorbent articles disclosed herein are illustrated in US 2016/0100999 (USSN 14/878,156), including Figs. 1-2 and 6-8; US 20l6/0270976 (USSN 62/136003), including the disposition of graphics 46a-f of Figs. 1-2, and 7-8; US 2008/0132872 (USSN 11/999,229). As disclosed by US 2008/0132872 (USSN 11/999,229), graphics of the present disclosure may be printed in part on the backsheet film and in part on the belt nonwovens. Further, articles of the present disclosure may include an anchoring graphic as described in US 2016/0100999 (USSN 14/878,156), filed on October 9, 2014. Each of the referenced graphics may be printed on the inner and outer surfaces of the belt nonwovens and chassis nonwoven and film as disclosed in US 2017/0290713 (USSN 62/319,463), filed on April 7, 2016, including the margins between graphics and components as disclosed in US 2017/0290713 (USSN 62/319,463).

As shown in Figure 2 the absorbent article 20 comprises an absorbent main body 38 to cover the crotch region of the wearer and a belt 40 extending transversely about the waist opening 36. The absorbent article 20 may also comprise an outer cover layer 42 to cover the main body 38. The belt 40 defines the waist opening 36. The belt 40, the main body 38 and/or the outer cover layer 42 jointly define the leg opening 34. One or more of the belt layers may extend from a first waist edge 134 in a first waist region 26 through the crotch region to a longitudinally opposing second waist edge 138 in a second waist region 28 and may form a portion or the whole of the outer surface of the absorbent article 20.

The absorbent main body 38 absorbs and contains body exudates disposed on the main body 38. The absorbent main body also comprises left and right longitudinally extending side edges 48 (hereinafter may be referred to as "longitudinal side edge") and front and back transversely extending end edges 50 (hereinafter may be referred to as "transverse end edge").

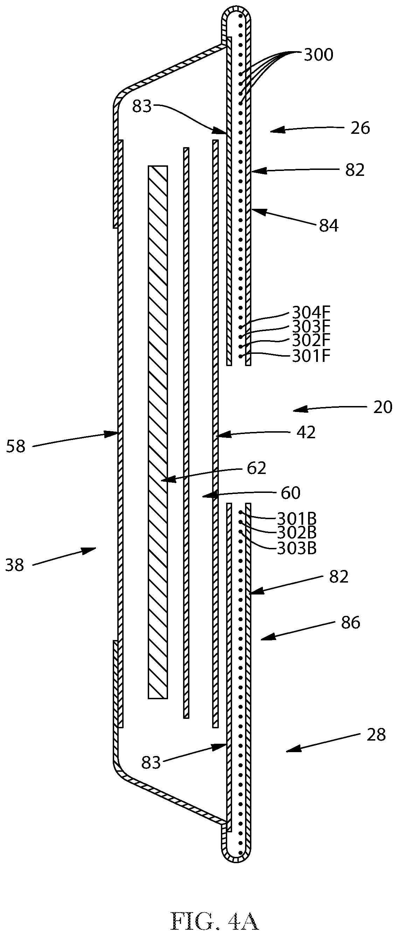

As shown in Figure 4A, the absorbent articles 20 may comprise front and rear belts 84, 86 intended to encircle at least a portion of the waist of the wearer, the front and rear belt portions 84, 86 being connected by a main body 38 forming the crotch region 30 of the absorbent article 20. The front and rear belts 84 and 86 may be formed from a first belt layer forming a portion of the outer surface 22 of the absorbent article, the first belt layer 82 (also referred to as an outer belt layer) may be formed of two longitudinally spaced webs of material. The front and rear belts 84 and 86 may also comprise a second belt layer (also referred to as an inner belt layer) 83 forming a portion of the inner surface 24 of the absorbent article 20, the second belt layer 83 may also be formed of two longitudinally spaced webs of material. The second belt layer 83 may also be discontinuous and spaced apart in a transverse direction. The first and second belt layers 82, 83 may be formed of substantially the same material or may comprise different materials. The first and second belt layers 82, 83 may be formed from nonwovens, films, foams, elastic nonwoven, or combinations thereof. The front and rear belts 84, 86 may also comprise an elastomeric material disposed between the first and second belt layers 82, 83. The elastomeric material may comprise one or more elastic strands, elastomeric films (including apertured films), elastomeric ribbons, elastomeric nonwovens, elastomeric filaments, elastomeric adhesives, elastomeric foams, scrims or combinations thereof. A portion of the elastomeric material may be directly combined with the outer cover layer. The main body 38 of the absorbent article may comprise an outer surface 22, backsheet 60, an inner surface 24, topsheet 58, and an absorbent core 62 disposed between the topsheet 58 and the backsheet 60. The backsheet may be formed of a nonwoven material, woven material, films or laminates comprising a combination of one or more of these materials. In one embodiment the backsheet is a film and nonwoven laminate wherein the nonwoven of the laminate is the outer cover layer. In addition, the main body 38 may comprise elasticized barrier leg cuffs 64 disposed at or adjacent the side edges of the main body. The front and rear belts 84, 86 may overlap at least a portion of the main body and one or both of the belt portions may be disposed on the outer surface of the main body or alternatively on the inner surface of the main body. A portion of the second belt layer and/or a portion of the first belt layer may be directly attached to the outer cover layer. Alternatively, the front belt and rear belt 84, 86 may comprise longitudinally spaced webs of material forming a first surface of the belt wherein the webs are folded along the waist edge, or alternatively the leg opening edge, of the belt to wrap the elastomeric material and form at least a portion of the second surface of the belt. In other words, at least a portion of the inner surface and outer surface of each of the belt portions may be formed from a single web of material. As shown in Fig. 4A, the first belt layer may form a portion of the garment facing surface, may fold over and be connected to the second belt layer 83, and may also be connected to a portion of the topsheet, such that the first belt layer also forms a portion of the wearer facing surface.

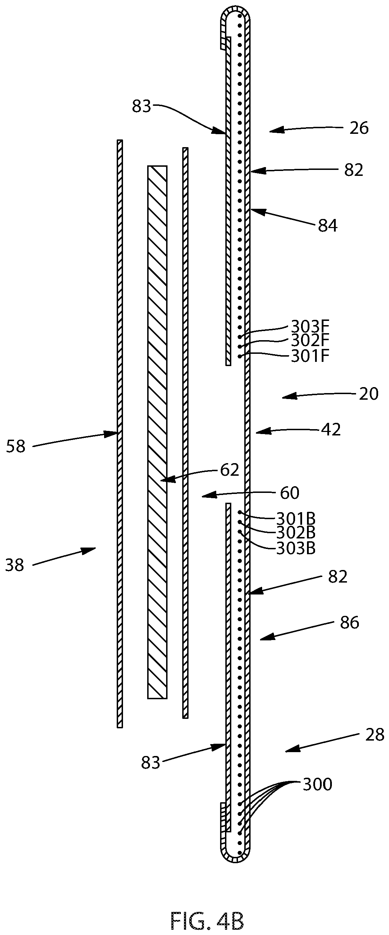

As shown in Figure 4B, the absorbent articles 20 may comprise front and rear elastic belts 84, 86 disposed in the front and rear waist regions 26, 28 respectively and intended to encircle at least a portion of the waist of the wearer, the front and rear belts 84, 86 being connected by the main body that forms the crotch region 30 of the article. The first and second belts may be formed from a first belt layer 82 extending from a first waist edge 134 in a first waist region 26 through the crotch region 30 to a longitudinally opposing second waist edge 138 in a second waist region 28 and forming a portion of the outer surface of the absorbent article 20. The front and rear belts 84, 86 also may comprise a second belt layer forming a portion of the inner surface 24 of the absorbent article, the second belt layer 83 may be formed of two longitudinally spaced webs of material. The first and second belt portions may also comprise an elastomeric material disposed between the first and second belt layers. The elastomeric material 300 may comprise elastic strands, elastomeric films (including apertured films), elastomeric ribbons, elastomeric nonwovens, elastomeric filaments, elastomeric adhesives, elastomeric foams, scrims or combinations thereof. The main body 38 of the absorbent article may comprise an outer surface 22, backsheet 60, an inner surface 24, topsheet 58, and an absorbent core 62 disposed between the topsheet 58 and the backsheet 60. The first belt layer may form a portion of the outer surface 22. In addition, the main body may comprise elasticized barrier leg cuffs 64 disposed at or adjacent the side edges of the main body. The second belt layer may overlap at least a portion of the main body and one or both of the second belt layer webs may form the outer surface of the first belt layer or alternatively the inner surface of the first belt layer. Alternatively, the front portion and/or the rear portion of the first belt layer may be folded along the waist edge of the belt region to wrap the elastomeric material and form a portion of the second belt layer of one or both of the front and rear belt portions 84, 86. In other words, the inner surface and outer surface of each of the belt portions is formed from a single web of material. As shown in Fig. 4B, the first belt layer 82 may form a portion of the garment facing surface, may fold over and be connected to the second belt layer 83, such that the first belt layer also forms a portion of the wearer facing surface.

The absorbent article may comprise curved leg elastics that run parallel to, and a few millimeters inboard of, at least a portion of the leg cut outs.

A portion or the whole of the main body 38 may be made extensible to a degree greater than the inherent extensibility of the material or materials from which the main body 38 is made, e.g., the backsheet 60. The additional extensibility may be desirable in order to allow the main body 38 to conform to the body of a wearer during movement by the wearer and or to provide adequate body coverage. The additional extensibility may also be desirable, for example, in order to allow the user of an absorbent article including a main body 38 having a particular size before extension to extend the front waist region 26, the back waist region 28, or both waist regions of the main body 38 to provide additional body coverage for wearers of differing size, i.e., to tailor the article to the individual wearer. Such extension of the waist region or regions may give the main body 38 a generally hourglass shape, so long as the crotch region is extended to a relatively lesser degree than the waist region or regions, and may impart a tailored appearance to the article when it is worn. In addition, the additional extensibility may be desirable in order to minimize the cost of the article 10. For example, an amount of material that would otherwise be sufficient only to make a relatively smaller article lacking this extensibility can be used to make an article capable of being extended to adequately cover a wearer that is larger than the unextended smaller absorbent article would fit.

A portion of the main body 38, for example, a portion of the chassis in one or both of the waist regions 26, 28 may be made laterally extensible to a maximum extensibility greater than a maximum extensibility of another portion of the main body 38 in the crotch region such that a lateral extension of each of the portions to its maximum extensibility imparts an hourglass shape to the main body 38. In one embodiment, the portion of the main body 38 underlying and/or immediately adjacent one or both of the front and back extensible belts may be made laterally extensible to a maximum extensibility greater than a maximum extensibility of another portion of the main body 38, for example the crotch region, such that a lateral extension of each of the portions to its maximum extensibility facilitates application of the absorbent article onto the body of a wearer by enabling the waist regions to be extended to fit over the wearer's hips and in addition, opening and orienting the leg openings enabling the wearer to place the legs through the openings more effectively.

Additional lateral extensibility in the main body 38 may be provided in a variety of ways. For example, a material or materials from which the main body 38 is made may be pleated by any of many known methods. Alternatively, all or a portion of the main body 38 may be made of a formed web material or a formed laminate of web materials like those described in U.S. Patent No. 5,518,801 (herein "Chappell et al.," issued on May 21,1996). This formed web material includes distinct laterally extending regions in which the original material has been altered by embossing or another method of deformation to create a pattern of generally longitudinally oriented alternating ridges and valleys and also includes laterally extending unaltered regions between the laterally extending altered regions. The formed web material can be extended in a direction perpendicular to the ridges up to the point where the ridges and valleys flatten with substantially less force than is required to extend beyond that point. In addition to lateral extensibility, the creation of a formed laminate web as described above provides a main body 38 backsheet with improved texture and cloth-like appearance and feel. The deformation creates a cloth-like pattern in the film and increases the loft of the nonwoven in multi-layer film and nonwoven laminate backsheets.

Alternatively, a portion of the absorbent article can be ring-rolled and thus rendered highly extensible as described in U.S. Patent No. 5,366,782 (herein "Curro et al.," issued on November 22, 1994). Specifically, a ring-rolling apparatus includes opposing rolls having intermeshing teeth that incrementally stretch and thereby plastically deform the material forming the absorbent article (or a portion thereof) thereby rendering the article extensible in the ring-rolled regions. In one embodiment, the absorbent article can be ring-rolled in a portion of at least one of the front or back waist regions, for example the portion of the main body 38 underlying and/or immediately adjacent one or both of the front and back belts 84, 86, while other regions may comprise a structured elastic-like formed web material. The article may be ring-rolled across the entire width in one or both of the waist regions or alternatively may be ring-rolled over only a portion of the main body 38 width or over only a portion of one or both of the belts.

The front laterally central portion and the back laterally central portion of the main body 38 may have a different range of extensibility from other portions of the main body 38. Additionally or alternatively, the laterally central portions may be extensible to a greater or lesser degree when subjected to a given level of opposing tensile forces, i.e., may be more easily or less easily extensible, than other portions of the main body 38.

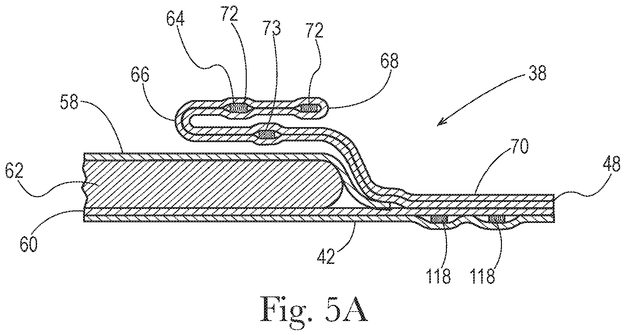

The main body 38 may comprise a liquid pervious topsheet 58, a liquid impervious backsheet 60 and an absorbent core 62 disposed therebetween. The main body 38 may additionally comprise a barrier leg cuff 64 disposed along the longitudinal side edge 48. The barrier leg cuff 64 provides improved containment of liquids and other body exudates in the crotch region 30. The barrier leg cuff 64 shown in Figure 5A comprises a single layer of material which may be folded to form a barrier leg cuff having two layers. The barrier leg cuff 64 extends from the side of the main body at or adjacent the longitudinal side edge 48 toward the longitudinal centerline L2. The barrier leg cuff may be folded along the folding line 66 back toward the longitudinal side edge 48. The barrier leg cuff 64 may have a first barrier cuff elastic material 72 adjacent to the distal portion 68 and a second barrier cuff elastic material 73 adjacent to the proximal portion 70 of the barrier leg cuff 64. The proximal portion 70 of the barrier leg cuff 64 may be joined to the backsheet 60 adjacent to the longitudinal side edge 48. The portion of the barrier leg cuff 64 along the folding line 66 and the distal portion 68 may be free from attachment to any portion of the main body 38 in the crotch region 30 such that the barrier leg cuff 64 stands up toward the wearer's body. The transverse end 74 of the barrier leg cuff 64 may be joined to the topsheet 58 at or adjacent the longitudinally opposing ends of the leg cuff by an attachment means which may be any known means such as an adhesive, heat bond, pressure bond or the like as shown in Fig. 5A. Other suitable leg cuffs are disclosed in US 2016/0270976 (USSN 62/136003) including those disclosed in Figs. 5A-C of 13744P.

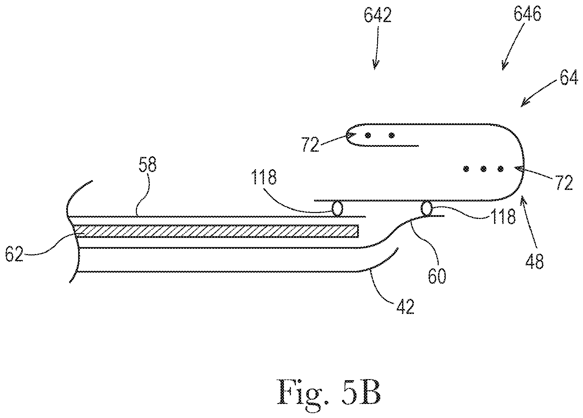

As shown in Fig. 5B, the cuff 64 (inner cuff 64a and outer cuff 64b) may have first, second, and third barrier cuff elastic materials 72a, b, and c; each of the elastic materials may be the same or different. A distal portion of the cuff 64 may be adhered to a distal portion of the backsheet film 60, and another portion of the cuff 64 may be adhered to the topsheet via adhesive 118. Beyond these cuff 64 configurations, other suitable examples of cuffs 64 that may be

5

used herein are disclosed in U.S. Patent No. 8,939,957 (herein "Raycheck et al.," granted on January 27, 2016), including the configurations disclosed by Figures 8a-t. The cuff 64 may be joined to the backsheet with a no leak bead 118' that runs along the entire longitudinal length of the cuff 64 and/or the backsheet film 60.

As shown in Fig. 5A, the backsheet film 60, the outercover nonwoven 42, and both layers of the cuff 64 may co-terminate at the side edge 48. Alternatively, as shown in Fig. 5B, the distal end of the cuff 64 may extend beyond the other materials to form at least a portion of the side edge 48 in a manner that exposes at least a portion of the cuff when the article 20 is worn, such that a more finished folded leg edge is achieved.

The liquid pervious topsheet 58 may be positioned adjacent the body-facing surface of the absorbent core 62 and may be joined thereto and/or to the backsheet 60 by any attachment means known in the art. The liquid impervious backsheet 60 is generally that portion of the absorbent article 20 positioned adjacent the garment-facing surface of the absorbent core 62 and prevents the exudates absorbed and contained therein from soiling articles that may contact the absorbent article 20. The absorbent core is positioned between the topsheet 58 and the backsheet 60 and absorbs and retains liquids such as urine and other certain body exudates.

The topsheet 58, the backsheet 60 and the absorbent core may be manufactured known materials. Suitable topsheet materials may include porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. Suitable backsheet materials may include breathable materials that permit vapors to escape from the diaper while still preventing exudates from passing through the backsheet.

A suitable absorbent core for use in the absorbent article 20 may comprise any absorbent material which is generally compressible, conformable, non-irritating to the wearer's skin, and capable of absorbing and retaining liquids such as urine and other certain body exudates. In addition, the configuration and construction of the absorbent core may also be varied (e.g., the absorbent core(s) or other absorbent structure(s) may have varying caliper zones, hydrophilic gradient(s), a superabsorbent gradient(s), or lower average density and lower average basis weight acquisition zones; or may comprise one or more layers or structures). In some embodiments, the absorbent core may comprise a fluid acquisition component, a fluid distribution component, and a fluid storage component. An example of a suitable absorbent core having a fluid acquisition component, a fluid distribution component, and a fluid storage component is described in U.S. Patent No. 6,590,136 (herein "Young et al.," granted on July 8, 2003).

Suitable absorbent cores of the present disclosure may comprise cellulosic airfelt material. For instance, such absorbent cores may comprise less than about 40%, 30%, 20%, 10%, 5%, or even 1% of the cellulosic airfelt material as determined by weight. Additionally, such an absorbent core may be primarily comprised of an absorbent gelling material in amounts of at least about 60%, 70%, 80%, 85%, 90%, 95%, or even about 100% as determined by weight. Furthermore, a portion of the absorbent core may comprise a microfiber glue (if applicable). Such absorbent cores, microfiber glues, and absorbent gelling materials are described in U.S. Patent No. 5,599,335 (herein "Goldman et al.," granted February 4, 1997); U.S. Patent No. 5,562,646 (herein "Goldman et al.," granted October 8, 1996); U.S. Patent No. 5,669,894 (herein "Goldman et al.," granted September 23,1997); U.S. Patent No. 6,790,798 (herein "Suzuki et al.," granted September 14,2004); U.S. Patent No.,521,587 (herein "Busam et al.," granted April 21, 2009); and in U.S. Patent Pub. No. 2004/0158212 (herein "Ponomarenko et al.," published August 12, 2004).

The absorbent core, including multiple layers making up the core system, may be printed and embossed as described in U.S. Patent No. 8,536,401 (herein "Ecker et al.," granted on September 17, 2013).

The absorbent core may be separable from the chassis as disclosed in U.S. Patent No. 6,989,006 (herein "LaVon et al.," granted on January 24, 2006; U.S. Patent No. 7,381,202 (herein "LaVon et al.," granted on June 3, 2008; U.S. Patent No. 7,175,613 (herein "Sugiyama et al.," granted on February 13, 2007; U.S. Patent No. 7,824,386 (herein "LaVon et al.," granted on November 2, 2010; U.S. Patent No. 7,766,887 (herein "Burns et al.," granted on August 3, 2010; and U.S. Patent No. 6,989,005 (herein "LaVon et al.," granted on January 24, 2006. In such embodiments, the measurements described in this disclosure may be made to the chassis alone or may be made to the chassis in combination with the separable core/absorbent assembly.

The absorbent article of the present disclosure, and particularly, a portion where the absorbent core is disposed, may have a body fluid absorption rate greater than 3 g/sec according to U.S. Patent No. 6,649,810 (herein "Minato et al.," granted on November 18, 2003). According to U.S. Patent No. 6,649,810 (herein "Minato et al.," granted on November 18, 2003), the expression "the portion (of the absorbent article) where the absorbent member is disposed" is intended to mean the portion occupied by the absorbent member when the absorbent article is flatly unfolded and seen in its plan view.

The absorbent core may have an intake factor greater than 3 according to U.S. Patent No. 7,073,373 (herein "La Fortune," granted July 11, 2006), wherein the intake factor is defined as the absorbent core permeability divided by the normalized retention capacity (which is defined by the Retention Capacity Test - also according to U.S. Patent No. 7,073,373 (herein "La Fortune," granted July 11, 2006)).

The absorbent core may have a body fluid absorption greater than 75 g/100 cm2, according to U.S. Patent No. 6,649,810 (herein "Minato et al.," granted on November 18, 2003).

A target location of the absorbent article may have a wicking value greater than 36%, according to U.S. Patent No. 6,383,960 (herein "Everett et al.," granted on May 7, 2002).

The absorbent article may have a bending stiffness between 0.05-1.0 gf, according to U.S. Patent No. 5,810,796 (herein "Kimura et al.," granted on September 22,1998).

The absorbent article may have a crotch fluid absorption rate greater than 3g/sec according to U.S. Patent No. 6,649,810 (herein "Minato et al.," granted on November 18, 2003). In one embodiment, a freeze-dried composite of the absorbent composite may have an intake rate of at least about 1.9 cubic centimeters (cc) of liquid/second at 80% composite saturation according to U.S. Patent No. 6,689,934 (herein "Dodge et al.," granted on February 10, 2004).

The absorbent core 200 may comprise channels as described in U.S. Patent No. 8,568,566 (herein Jackels et al.," granted on October 29, 2013; U.S. Pub. No. 2012/316046 (herein "Jackels et al.," published on December 13, 2012; U.S. Pub. No. 2014/027066; U.S. Patent No. 8,979,815 (herein "Roe et al.," granted on March 17, 2015); U.S. Patent No. 9,216,118 (herein "Roe et al.," granted on December 22, 2015); U.S. Patent No. 9,216,116 (herein "Roe et al.," granted on December 22, 2015); U.S. Pub. No. 2012/316526 (herein "Rosati et al.," published on December 13, 2012); U.S. Pub. No. 2012/316527 (herein "Rosati et al.," published on December 13, 2012); U.S. Pub. No. 2012/316528 (herein "Kreuzer et al.," published on December 13, 2012); U.S. Pub. No. 2012/316529 (herein "Kreuzer et al.," published on December 13, 2012); U.S. Pub. No. 2012/316523 (herein "Hippe et al.," published on June 30, 2015); U.S. Pub. No. 2014/163501 (herein "Ehrnsperger et al.," published on June 12, 2014); U.S. Pub. No. 2014/163502 (herein "Arizti et al.," published on June 12, 2014); U.S. Pub. No. 2014/163503 (herein "Arizti et al.," published on June 12, 2014); and European Pub. Nos. 2532328, 2532329, 2717823, 2717820, 2717821, 2717822, 2532332, 2740449, and 2740452.

The outer cover layer 42 may be disposed on the outer surface 22 of the absorbent article 20 and may cover the crotch panel 56 of the absorbent main body 38. The outer cover layer 42 may extend into and cover the front waist panel 52 and the back waist panel 54 of the main body 38. The outer cover layer may form a portion of the backsheet and/or the main body. The outer cover layer 42 may be directly joined to and cover a portion or all of the liquid impervious backsheet 60 of the main body 38. The central panel 80 of the front and back belt 84, 86 may be joined to the front waist panel 52 and the back waist panel 54 of the main body 38 through the outer cover layer 42. Thus, the outer cover layer 42 may be disposed between the front and back belt 84, 86 and the liquid impervious backsheet 60 of the main body 38. In one embodiment, the outer cover layer 42 may be coextensive with the liquid impervious backsheet 60. The leg elastic material 140 is disposed so as to extend generally longitudinally along the longitudinal side edge 48 of the main body 38. The leg elastic material 140 may be disposed at least in the crotch region 30 of the absorbent article 20 or may be disposed along the entirety of the longitudinal side edge 48.

The outer cover layer 42 may comprise a material separate from the material of the inner layer 83 and the outer layer 82 constituting the belt 40. The outer cover layer 42 may comprise two or more layers of materials. The outer cover layer 42 may comprise any known materials and may comprise materials used for the front and back belt 84, 86 as explained above. The outer cover layer 42 may comprise a single layer of nonwoven web of synthetic fibers. The outer cover layer 42 may comprise a single layer of hydrophobic, non-stretchable nonwoven material. The outer cover layer may comprise a film, a foam, a nonwoven, a woven material or the like and/or combinations thereof such as a laminate of a film and a nonwoven.

The belt 40 comprises a front belt 84 and a back belt 86 (hereinafter may be referred to as "front and back belt 84, 86") and has a ring-like configuration by permanently or refastenably connecting the front belt 84 and the back belt 86 at the seams 32. The back belt may have a greater longitudinal extent than the front belt in the seam region such that the back belt has an appendix region 500 (see Figs. 1, 2, and 3A) that extends beyond the seams 32. The appendix region may be 15-80 mm. Suitable designs for the appendix region are disclosed in U.S. Patent No. 7,901,393 (herein "Matsuda et al.," granted on March 8, 2011.

The belt 40 may be ring-like and elastic. The ring-like elastic belt 40 extends transversely about the waist opening 36 of the absorbent article 20 and acts to dynamically create fitment forces and to distribute the forces dynamically generated during wear. Applicants have found that improved fit can be created by controlling the distance between, linear density, and the pre-strain of the elastomeric material in relation to each other and to the openings for the body. This may occur by choosing different materials throughout the belt 40 that exhibit desired properties. The different materials are combined at specific distances, linear densities, and prestrains to create a belt 40 that acts to dynamically create fitment forces. This improved fit translates into reduced sagging and or gapping problems around the waist opening. The examples below illustrate various properties of the different portions of the belt embodiments that are suitable:

The data in the example tables below is hypothetical and based on the Test Methods Section described in this application for i) Average Elastic Spacing, S, ii) Average Dtex, Dt, iii) Average pre-strain, PS, and iv) Average Force at 50% strain, F50. Each of Examples 1-3 illustrate inventive pant-type disposable absorbent articles:

**Example 1**

| Elastic Sections | Average Pre-Strain, PS (%) | Average Dtex, Dt | Average Force at 50% Strain, F5O, N/cm | Average Spacing, S, mm |
|---|---|---|---|---|
| First (102) | 90-160 | 450-700 | 0.2-0.5 | 3-6 |
| Second (104) | 90-160 | 400-600 | 0.1-0.4 | 3-6 |
| Third (106) | 90-160 | 400-600 | 0.1-0.4 | 3-6 |
| Fourth (108) | 90-160 | 450-700 | 0.2-0.5 | 3-6 |
| Fifth (100) | | | | |
| Fifth - A (100A) | 60-160 | 400-600 | 0.02-0.20 | 4-10 |
| Fifth - B (100B) | | | | |
| Sixth (101) | | | | |
| Sixth - A (101A) | 60-160 | 400-600 | 0-0.10 | 5-30 |
| Sixth - B (101B) | 60-160 | 400-600 | 0.02-0.20 | 5-20 |
| *Assumes no set in the elastics | | | | |

**Example 2**

| Elastic Sections | Average Pre-Strain (%) | Average Dtex | No. of elastics | Distance 1st to last elastic | Average Spacing | Width of Elastic Section, mm | Average Force at 50 % Strain, N/cm | Average Force at 75% Strain | Average Force at 100% Strain |
|---|---|---|---|---|---|---|---|---|---|
| First (102) | 150 | 560 | 22 | 84 | 4 | 89 | 0.32 | 0.43 | 0.55 |
| Second (104) | 150 | 470 | 22 | 84 | 4 | 89 | 0.27 | 0.36 | 0.46 |
| Third (106) | 150 | 470 | 22 | 84 | 4 | 89 | 0.27 | 0.36 | 0.46 |
| Fourth (108) | 150 | 560 | 22 | 84 | 4 | 89 | 0.32 | 0.43 | 0.55 |
| Fifth (100) | | | | | | | | | |
| Fifth - A (100A) | 150 | 470 | 3 | 13 | 6.5 | 76 | 0.04 | 0.06 | 0.07 |
| Fifth - B (100B) | 0 | 470 | 0 | | | 76 | 0.00 | 0.00 | 0.00 |
| Sixth (101) | | | | | | | | | |
| Sixth - A (101A) | 0 | | 1 | | | 76 | | | |
| Sixth - B (101B) | 150 | 470 | 7 | 59 | 9.8 | 76.0 | 0.10 | 0.13 | 0.17 |

*Assumes no set in the elastics

**Example 3**

| Elastic Sections | Average Pre-Strain (%) | Average Dtex | No. of elastics | Distance 1st to last elastic | Average Spacing | Width of Elastic Sectio n, mm | Average Force at 50% Strain, N/cm | Average Force at 75% Strain, N/cm | Average Force at 100% Strain, N/cm |
|---|---|---|---|---|---|---|---|---|---|
| First (102) | 124 | 560 | 22 | 84 | 4 | 89 | 0.28 | 0.38 | 0.48 |
| Second (104) | 110 | 470 | 22 | 84 | 4 | 89 | 0.24 | 0.32 | 0.40 |
| Third (106) | 110 | 470 | 22 | 84 | 4 | 89 | 0.24 | 0.32 | 0.40 |
| Fourth (108) | 124 | 560 | 22 | 84 | 4 | 89 | 0.28 | 0.38 | 0.48 |
| Fifth (100) | | | | | | | | | |
| Fifth - A (100A) | 110 | 470 | 7 | 24 | 4 | 76 | 0.09 | 0.12 | 0.15 |
| Fifth - B (100B) | 0 | | 0 | | | 76 | 0.00 | 0.00 | 0.00 |
| Sixth (101) | | | | | | | | | |
| Sixth - A (101A) | 90 | 470 | 4 | 30 | 10 | 76 | 0.05 | 0.07 | 0.08 |
| Sixth - B (101B) | 90 | 470 | 10 | 70 | 7.8 | 76 | 0.13 | 0.17 | 0.21 |
| *Assumes no set in the elastics | | | | | | | | | |

Each of the elastics in the front and back belts, as well as the crotch region, may have the same dtex, Dt, (e.g., 800) and the same pre-strain, PS, (e.g., 120%). Another suitable embodiment may have each of the elastics of the front and back belts at 470 dtex at about 120 to 150% strain, with lower dtex (smaller diameter of strands) and/or lower pre-strain (less pre-strain) in the front and/or back regions of the crotch region. The front and back belt 84, 86 may comprise any known materials. Suitable material for the front and back belt 84, 86 can be manufactured from a wide range of materials such as plastic films; apertured plastic films; woven or nonwoven webs of natural materials (e.g., wood or cotton fibers); synthetic fibers (e.g., polyolefins, polyamides, polyester, polyethylene, or polypropylene fibers), or a combination of natural and/or synthetic fibers; or coated woven or nonwoven webs. The belt may comprise a nonwoven web of synthetic fibers. The belt may comprise a stretchable nonwoven. The belt may comprise an inner hydrophobic, non-stretchable nonwoven material and an outer hydrophobic, non-stretchable nonwoven material.

The belt 40 may comprise a first elastic section 102 (e.g., a first plurality of elastics) and a second elastic section 104 (e.g., a second plurality of elastics) located in the front belt 84. These two sections may be equally divided by longitudinal distance along the seam or equally divided from the waist end edge to the leg opening. The belt 40 may comprise a third elastic section 106 (e.g., a third plurality of elastics) and a fourth elastic section 108 (e.g., a fourth plurality of elastics) located in the back belt 86. These two sections may be equally divided by longitudinal distance along the seam or from the waist end edge to the leg opening. Fifth and sixth elastic sections 100 and 101 (having a fifth and a sixth plurality of elastics) may be disposed distal (i.e., toward the transverse axis T1) of each front and back waist regions 26 and 28. The fifth and sixth elastic sections 100 and 101 may be equally divided by longitudinal distance along the crotch region or longitudinally from the leg opening in the front to the leg opening in the back. Fifth elastic section 100 may be further equally divided by longitudinal distance to form subsections 100A and 100B. Also, sixth elastic section 101 may be further equally divided by longitudinal distance to form subsections 101A and 101B. The first elastic section 102 and the fourth elastic section 108 are adjacent to the waist opening 36. The second elastic section 104 and the third elastic section 106 are adjacent to the leg openings 34.

According to the method of defining elastic sections according to the Test Methods Section described in this application, the first elastic section 102 may comprise 20 percent to 80 percent, 25 percent, 40 percent, 50 percent, 60 percent, 70 percent of the longitudinal direction length of the front belt 84. The second elastic section 104 may comprise of 20 percent to 80 percent, 25 percent, 40 percent, 50 percent, 60 percent, 70 percent of the longitudinal direction length of the front belt 84. The third elastic section 106 may comprise 20 percent to 80 percent, 25 percent, 40 percent, 50 percent, 60 percent, 70 percent of the longitudinal direction length of the back belt 86. The fourth elastic section 108 may comprise 20 percent to 80 percent, 25 percent, 40 percent, 50 percent, 60 percent, 70 percent of the longitudinal direction length of the back belt 86.

The belt 40 may comprise a front border between the first elastic section 102 and a second elastic section 104, and the front border may be located within 5mm, 10mm, 20mm, 30mm 40mm, 50mm from the front edge of the absorbent core. The belt 40 may comprise a back border between the third elastic section 106 and a fourth elastic section 108, and the back border may be located within 5mm, 10mm, 20mm, 30mm 40mm, 50mm from the back edge of the absorbent core.

The belt 40 may comprise a first force zone 110, a second force zone 112, a third force zone 114, a fourth force zone 116, a fifth force zone 118, and a sixth force zone 120 located in the front belt 84. The first force zone 110, second force zone 112, and third force zone 114 may be located in the first elastic section 102. The fourth force zone 116, fifth force zone 118, and sixth force zone 120 may be located in the second elastic section 104.

The belt 40 may comprise a seventh force zone 122, an eight force zone 124, a ninth force zone 126, a tenth force zone 128, a eleventh force zone 130, and a twelfth force zone 132 located in the back belt 86. The seventh force zone 122, eight force zone 124, and ninth force zone 126 may be located in the third elastic section 106. The tenth force zone 128, eleventh force zone 130, and twelfth force zone 132 may be located in the fourth elastic section 108.

The thirteenth force zone 334, fourteenth force zone 336, fifteenth force zone 338, and sixteenth force zone 340 may be located in the fifth elastic section 100 in the front region of the crotch region. The seventeenth force zone 342, eighteenth force zone 344, nineteenth force zone 346, and twentieth force zone 348 may be located in the sixth elastic section 101 in the back region of the crotch region.

Force zones may be equally distanced throughout the belt along the longitudinal axis in the front and back belts 84, 86 and may each comprise a transverse force of 1 to 10N/zone measured at a strain of 50%. Force zones may also be unequally distanced throughout the belt along the longitudinal axis in the front and back belts 84, 86. Force zones may have varying width and length. Force zones may be continuous or discontinuous, as for example, when disrupted by the main body 38 and/or absorbent core.

According to the method of defining elastic sections according to the Test Methods Section described in this application, the front belt 84 may comprise 5 to 70 or 10 to 60 elastic strands. The back belt 86 may comprise 5 to 70 or 10 to 60 elastic strands. The front crotch region may comprise 1 to 10 or 2 to 6 elastic strands. The back crotch may comprise 3 to 30 or 5 to 20 elastic strands. The elastic strands may be distributed amongst the different force zones. Elastic strands may be distributed evenly amongst the force zones. Elastic strands may also be distributed unevenly amongst the different force zones.

The elastic strands 300 may have a linear density between 200 to 2500. Linear density is the density of the elastic fibers in the elastic strand. The most commonly used unit for the linear density is the decitex, abbreviated dtex, which is the mass in grams per 10,000 meters (Dt). The linear density may be used to change the force profile. For example, one could reach a desired force profile by selecting the linear density of a single elastic strand, combining multiple elastic strands with a smaller linear density in close proximity to each other, and/or combining with other elastomeric materials.

The elastic strands may have an elastic pre-strain. The elastic pre-strain is the percent of length increase in an elastic strand or plurality of elastic strands at the point of combining the elastic(s) with the first and/or second belt layers. For example a strand with a free length of 15 centimeters (cm) may have a load applied such that the 15 cm elastic strand is now 18 cm long. This length increase of 3 cm is 20% of 15 cm (3/15), or a 20% strain. The elastic pre-strain may be used to change the force profile of a single elastic strand or a plurality of elastic strands. Force profiles may also be changed by changing the linear density in conjunction with the elastic pre-strain of one or more elastic strands.

According to the method of defining elastic sections according to the Test Methods Section described in this application, the elastic pre-strain of the first elastic section may be higher than the elastic pre-strain of the second elastic section. Similarly, the elastic pre-strain of the fourth elastic section may be higher than the elastic pre-strain of the third elastic section. This is accomplished by using higher pre-strain in elastics that are close to the first waist edge (also called the front waist edge) and the second waist edge (also called the back waist edge). This makes the circumference of the absorbent article smaller at the waist edge compared to the rest of the belt region, giving the absorbent article a more underwear like look.

According to the method of defining elastic sections according to the Test Methods Section described in this application, the elastic pre-strain of the eighth force zone 124 found in the third elastic section 106 of the back belt 86 may be greater than, equal to, or less than the elastic pre-strain of both the seventh force zone 122 and the ninth force zone 126. The elastic pre-strain of the eleventh force zone 130 found in the fourth elastic section 108 of the back belt 86 may be greater than, equal to, or less than the elastic pre-strain of both the tenth force zone 128 and the twelfth force zone 132.

According to the method of defining elastic sections according to the Test Methods Section described in this application, the linear density of the elastic in the first elastic section may be greater than the linear density in the second elastic section. Similarly, the linear density of the elastic in the fourth elastic section may be greater than the linear density in the third elastic section. This delivers a higher force at the waist edge compared to the rest of the belt, which is desired to deliver underwearlike fit. The force of the first elastic section 102 may not be equal to the second elastic section 104. The force of the third elastic section 106 may not be equal to the fourth elastic section 108. Elastics located in the fourth elastic section 108 transversely cover substantially the whole section continuously. Elastics located in the third elastic section 106 may be laterally interrupted by the main body 38 and/or absorbent core.

The number of elastic strands in each zone may be changed according to the placement of the absorbent core. Applicants have found that the use of thinner absorbent cores may lead to a need in increased elastic force to compensate for the change in article thickness. The force profile must be adjusted depending on the location and thickness of the absorbent core. This particularly affects the second, third, fifth and sixth elastic sections.

The elastic strands disposed in the belt may be aligned in a curved fashion so that a tangent of the curve of the elastic strands may form an acute angle with the centerline or may form an arcuate shape. This may allow for targeting the force profile and/or coordinating print and elastication/rugosities/elastics in the stretch sections.

The gaps between the elastic strands 300 may be 2 mm, 3mm, 5 mm, 6mm, 7mm, 8mm, 9mm, 10mm, 11mm, 12mm, 13mm, 14mm, 15mm, 16 mm, 17 mm, 18 mm, 19 mm, 20mm, 21mm, or 22 mm, or 25 mm.

As shown in Figs. 3B and 3C, spacers 136 (e.g., 136a-h) may be used in elastic sections disposed adjacent to the leg openings (e.g., sections 104 and 106), as well as in the crotch region (sections 100 and 101). The spacers 136 may be at least 1.1, 1.2, 1.3, 1.4, 1.5, 1.6, 1.7, 1.8, 1.9, 2, 2.5, 3, 4, or 5 times greater than the average spacing of the elastics disposed in certain sections. Spacers may be 1 mm, 2mm, 3 mm, 3.5 mm, 4 mm, 4.5 mm, 5 mm to 25 mm, 7mm to 20 mm, 10 mm to 15 mm, and/or 12 mm to 13 mm in the longitudinal direction.

The embodiment of Fig. 3B illustrates spacers (136a-c) disposed between the front belt 84 (such that spacer 136a is from about 10-40 mm below the distal edge of the front belt 84). Each of spacers 136a-c may be greater than the gaps provided in Section 104 (the bottom half of the front belt) and/or Section 102 (the top half of the front belt). Further, spacer 136b (i.e., the distance between the second most distal elastic 302F and the third most distal elastic 303F in the front region 27) may be greater in longitudinal distance than spacer 136c (i.e., the distance between the third most distal elastic 303F and the fourth most distal elastic 304F in the front region 27), and spacer 136a (i.e., the distance between the first most distal elastic 301F and the second most distal elastic 302F in the front region 27) may be greater in longitudinal distance than spacer 136b. It should be understood that in alternative embodiments, spacers may be disposed in Section 104 in the front belt. In alternative embodiments, spacer 136c may be from 5-7 mm, spacer 136b may be from 6-8 mm, and spacer 136a may be from 7-9mm.

Further referring to Fig. 3B, spacers (136d-h) may be disposed between the back belt 86 (such that spacer 136h is from about 10-40 mm below the distal edge of the back belt 86). Each of spacers 136d-h may be greater than the gaps provided in Section 108 (the top half of the back belt) and/or Section 106 (the bottom half of the back belt). Further, spacer 136d (i.e., the distance between the first most distal elastic 301B and the second most distal elastic 302B in the back region 29) may be greater in longitudinal distance than spacer 136e (i.e., the distance between the second most distal elastic 302B and the third most distal elastic 303B in the back region 29), and spacer 136e may be greater in longitudinal distance than spacer 136f (i.e., the distance between the third most distal elastic 303B and the fourth most distal elastic 304B in the back region), spacer 136f may be greater in longitudinal distance than spacer 136g (i.e., the distance between the fourth most distal elastic 304B and the fifth most distal elastic 305B in the back region), spacer 136g may be greater in longitudinal distance than spacer 136h (i.e., the distance between the fifth most distal elastic 306B and the sixth most distal elastic 306B in the back region). It should be understood that in alternative embodiments, spacers may be disposed in Section 106 in the back belt. In alternative embodiments, spacer 136h may be from 5-7 mm, spacer 136g may be from 6-8 mm, and spacer 136f may be from 7-9mm, spacer 136e may be from 8-10 mm, and spacer 136d may be from 9-11 mm.

The embodiment of Fig. 3C has 4 mm gaps between the elastics in Sections A and B. Section A extends longitudinally from a front waist end edge 134 of the article to a position from about 5-30 mm past a distal edge 226 of the front waist region 26. Section B extends longitudinally from a back waist end edge 138 of the article to a position from about 15-80 mm past a distal edge 228 of the back waist region 28. The last several elastics below the front belt 84 are spaced apart greater than 4 mm, such that spacer 136b (the second most distal spacer) is 5 mm and spacer 136a (the first most distal spacer) is 6 mm. The last several elastics below the back belt 86 are also spaced apart greater than 4 mm, such that spacer 136f (the fourth most distal spacer in the back region) is 5 mm, spacer 136e (the third most distal spacer in the back region) is 6 mm, spacer 136d (the second most distal spacer in the back region)is 7 mm, and spacer 136c (the first most distal spacer in the back region) is 8 mm. Fig. 3C also illustrates that a portion of the leg openings may be convexly shaped 400.

As shown in Fig. 3D, the absorbent article may comprise a mega-gap 200 in the crotch region that is from about 15 mm to about 90 mm, from about 25 mm to about 75 mm, or from about 35 mm to about 50 mm. All or a majority of the mega-gap 200 may be forward of the transverse centerline T1 (i.e., in the front region 27), such that 200F may be from about 15 mm to about 90 mm, from about 25 mm to about 75 mm, or from about 35 mm to about 50 mm. Likewise, all or a majority of the mega-gap 200 may be backward of the transverse centerline T1 (i.e., in the back region 29), such that 200B may be from about 15 mm to about 90 mm, from about 25 mm to about 75 mm, or from about 35 mm to about 50 mm. The mega-gap 200 may function to provide an area that does not have any transversely disposed elastics creating transverse stresses on the article, such that the portion of the core overlapping with the mega-gap does not bunch. Another benefit of the mega-gap is preventing the longitudinally disposed leg cuffs from distorting and compromising the seal of leg cuffs against the wearer's skin. This may be especially important for articles comprising inner barrier leg cuffs.

PACKAGES

The absorbent articles of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

Accordingly, packages of the absorbent articles of the present disclosure may have an In-Bag Stack Height of less than about 100 mm, less than about 95 mm, less than about 90 mm, less than about 85 mm, less than about 85 mm, but greater than about 75 mm, less than about 80 mm, less than about 78 mm, less than about 76 mm, or less than about 74 mm, specifically reciting all 0.1 mm increments within the specified ranges and all ranges formed therein or thereby, according to the In-Bag Stack Height Test described herein. Alternatively, packages of the absorbent articles of the present disclosure may have an In-Bag Stack Height of from about 70 mm to about 100 mm, from about 70 mm to about 95 mm, from about 72 mm to about 85 mm, from about 72 mm to about 80 mm, or from about 74 mm to about 78 mm, specifically reciting all 0.1 mm increments within the specified ranges and all ranges formed therein or thereby, according to the In-Back Stack Height Test described herein.

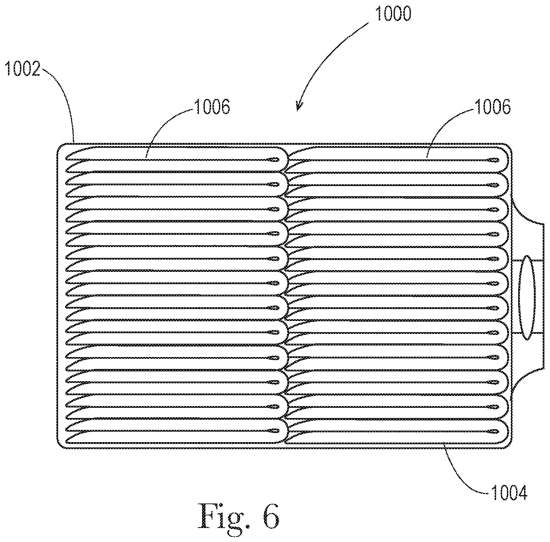

Fig. 6 illustrates an example package 1000 comprising a plurality of absorbent articles 1004. The package 1000 defines an interior space 1002 in which the plurality of absorbent articles 1004 are situated. The plurality of absorbent articles 1004 are arranged in one or more stacks 1006.

TEST METHODS SECTION

These tests can be used for stretch laminates that comprise elastic strands, e.g. Spandex strands. Typically, the elastic strands are stretched and laminated between two nonwovens to make the stretch laminate. The intent of these tests is to characterize these stretch laminates in terms of i) the strand spacing, i.e. the distance from one strand to the next and ii) their dtex (or decitex). Since these tests will be done for each elastic section, it represents an average value for all the elastics in that section. These tests are suitable for pant type absorbent articles, comprising parallel strand elastics that run predominantly in the transverse direction of the product.

Sample Prep

The purpose of this step is to separate the belt 40, which comprises the elastic material 300, from the main body 38, which comprises the absorbent core 62. Place the absorbent article 20, also referred to as "product," on the tabletop with the front side facing up. The product is laid flat on the table with the side seams intact. The product is spread out as much as possible, while still being in a relaxed state. With a marker, mark the location of the lower ends of the side seams, on the outer side of the front and the back belts; i.e. make a total of 4 marks (dots). Draw the longitudinal centerline, Li, from the first waist edge 134 (also referred to as "front waist edge") to the second waist edge 138 (also referred to as "back waist edge"). Draw the transverse centerline, T1. Next, draw a straight line, T3, connecting the lower ends of the side seam in the front, from the left side seam to the right side seam. Turn the product over so the back side of the product is facing up. Draw a straight line, T6, connecting the lower ends of the side seam in the back, from the left side seam to the right side seam. The front waist edge, the back waist edge and lines T3, T1 and T6 divide the absorbent article in 4 parts.

Turn the product over so the front of the product is facing up. Draw line T2, parallel to line T3 and halfway between the front waist edge and T3, to form the First Elastic Section (102 in the spec) and the Second Elastic Section (104 in the spec) as shown in Figure 3A. Draw a line T4, parallel to T3 and half way between T3 and T1, to form the Fifth Elastic Section--A (100a in the spec) and Fifth Elastic Section--B (100b in the spec) as shown in Figure 3A.

Repeat these steps on the back. Draw a line T5, parallel to T1 and half way between T1 and T6, to form the Sixth Elastic Section--A (101a in the spec) and Sixth Elastic Section--B (101b in the spec) as shown in Figure 3A. Finally, draw a line T7 parallel to T6 and half way between T6 and the back waist edge, to form Third Elastic Section (106 in the spec) and Fourth Elastic Section (108 in the spec) as shown in Figure 3A.

Mark each of the eight Elastic Sections appropriately. Open up the product by cutting the sides open (at the side seams). Remove the main body from the belt. In most cases, it is a glued bond, which can be opened up using Freezelt. Cut along the transverse lines T2, T3, T4, T1, T5, T6 and T7 to obtain the 8 Elastic Sections. If any transverse line falls exactly on an elastic, make the cut about a millimeter past that elastic, moving closer towards the transverse center line T1.

If the belt 40 has at least one nonwoven that extends the full length and width of the product, there will be all 8 Elastic Sections. If, on the other hand, the belt 40 does not have a nonwoven that extends the full length and width of the product, it is possible that there may be less than 8 Elastic Sections, depending on the width of the belt. If any of the Sections has no elastic, no measurements need to be made on it. If the belt has some curved leg elastics, do not use those elastics.

In each of the 8 Elastic Sections, measure the following. For each variable, make 5 replicate measurements (n=5).

**A) Average Elastics Spacing, S**

Start with the First Elastic Section. Identify the area of this Elastic Section that has elastics. If there is a gap of over 30 mm (in the longitudinal direction, parallel to Li) between adjacent elastics, then these elastics should be considered to be in two different areas within that section. If the elastic spacing is 30 mm or less, it should be counted as being in the same area. Count the number of elastics in an area. Measure the distance from the first elastic to the last elastic in an area (xi). Count the number of elastics in that area (ni). Repeat for all the areas with elastics within each Elastic Section (2, 3,). Calculate the Average Elastic Spacing for the First Elastic Section, S-first, as follows:

$$\text{S-first} = (x_1+x_2+x_3+...)/\left[(n_1 - 1) + (n_2 - 1) + (n_3 - 1) +...\right]$$

where subscript 1 is for the first area, subscript 2 is for the second area, etc. within this First Elastic Section. Subscript -first is for the First Elastic Section.

Repeat this for all the 8 Elastic Sections (S-second, S-third,...., S-sixthB).

**B) Average Elastics Dtex, Dt**

The elastics may either be glued to the nonwoven or not. If the elastics are not glued to the nonwovens and are placed in the laminate as drawstring elastics, remove all the elastics from each Elastic Section of the laminate and place them separately making sure that they are labelled appropriately. No further treatment is needed on these elastics. If, on the other hand, the elastics are glued to the nonwovens, remove all the elastics by using Freezeit. Once again, ensure that the elastics from each Elastic Section are labelled appropriately and placed separately. Since these elastics have glue on them, use an appropriate solvent to dissolve the glue without affecting the elastics. For example, if the elastics are made of polyurethane (Spandex solvent may be used). Evaporate the solvent.

With the elastics removed and also freed of the glue, measure the total relaxed length of all the elastics in the First Elastic Section (L1, L2,...) in mm. Next, weigh all the elastics together in the First Elastic Section (W-first) in grams.

Calculate the Average Elastics Dtex in the First Elastic Section, Dt-first, as follows:

$$\text{Dt-first} = (\text{W-first})/(\text{L1}+\text{L2}+\text{L3}+...)*10{,}000{,}000.$$

Repeat this measurement for the remaining 7 Elastic Sections (Dt-second, ..., Dt-sixthA, Dt-sixthB).

**C) Average % Pre-strain, PS**

Starting with a new product sample, i) mark the 8 Elastic Sections, ii) separate the belt 40 from the main body, and iii) cut off the 8 Elastic Sections as per the Sample Prep description in the previous test for the Average Elastics Spacing and the Average Elastics Dtex.

Take the First Elastic Section and stretch it out fully on the tabletop using masking tape to hold the sides. In this test, the stretch direction is referred to as the length direction and the direction perpendicular to the stretch direction is referred to as the width direction of the Elastic Section. It should be noted that, for this test, and also for the tensile test later on, the length direction is perpendicular to the longitudinal center line, Li, of the absorbent article, and the width direction is parallel to the longitudinal center line Li. Draw 2 vertical lines that are parallel to the longitudinal center line L1 and are at a distance of 100 mm on either side of the longitudinal center line Li. These two lines should extend the full width of the Elastic Section. This defines the fully stretched length, Lmax-first, which is 200 mm for this First Elastic Section. If the Elastic Section is not long enough to get the 200 mm long stretched sample, a shorter length may be used. This shorter length should be ~40 mm less than the minimum length of the fully stretched Elastic Section in order to leave some room to attach the masking tape at the two sides. This shorter length is the fully stretched length, Lmax-first, for the narrower Elastic Section.

With an Exacto knife, cut off all the elastics and the nonwovens along these two lines. Keep the center portion of this Elastic section for further analysis.

Separate the elastics from the nonwovens using Freezeit. Count the number of elastics, n-first, in this First Elastic Section. If the elastics have glue on them, dissolve the glue in an appropriate solvent that does not significantly affect the elastics (e.g. Tetra hydro furan, THF, for polyurethane strands). Allow the elastics to dry out.

Measure the total relaxed length, Lr-first, of all the elastics in this First Elastic Section.

Calculate Average % pre-strain, PS-first, as follows:

$$\text{PS-first} = (\text{Lmax-first} * \text{n-first} - \text{Lr-first})/\text{Lr-first} * 100$$

Repeat this measure for each of the 7 remaining Elastic Sections (PS-second, ..., PS-sixthB).

**D) Elastic Force, F**

Starting with a new product sample, i) mark the 8 Elastic Sections, ii) separate the belt 40 from the main body 38, and iii) cut off the 8 Elastic Sections as per the Sample Prep description in the previous test for the Average Elastics Spacing and the Average Elastics Dtex.

Take the First Elastic Section and lay it down on the table in a relaxed state. Gently spread out the sample so it lies uniformly, while still being in a relaxed state. As in the test for measuring pre-strain, the stretch direction is referred to as the length direction and the direction perpendicular to the stretch direction is referred to the width direction for this test.

Draw 2 vertical lines that are parallel to the longitudinal center line, Li, and are at a distance of 50 mm on either side of the longitudinal center line, Li. These two lines should extend the full width of the Elastic Section. If this Elastic Section is not long enough to get this 100 mm long relaxed sample, a smaller length may be used. This smaller length should be ~20 mm less than the minimum length of this Elastic Section.

Measure the width of this First Elastic Section, Ws-first.

Measure the tensile properties of this stretch laminate in a tensile tester as described below.

1) On an Instron or an MTS tensile tester, set the program to measure force as a function of strain.

2) Set the initial gage length to 50 mm. If it is not possible to get 50 mm gage length, use the maximum gage length possible.

3) Set the crosshead speed to 10 in./min.

4) Place the sample in between grips of the tensile tester, ensuring that the grips are wider than the sample.

5) Clamp the sample in the top grip and zero the load cell.

6) Clamp the sample in the bottom grip, while ensuring that there is some slack in the sample. The sample should not be under tension at this time.

7) Start the test. The tensile tester will collect load vs. displacement data. Record the distance between the grips when the force is 0.05 N. This is the adjusted gage length corresponding to zero strain. It is sometimes referred to as slack preload.

8) Record load vs. strain using the adjusted gage length as the starting length.

9) Continue collecting data until a strain of 100% is reached.

10) Report load in Newtons/cm at 50, 75 and 100% strains (F50-first, F75-first, and F100-first) using the width of the sample, Ws-first.

Repeat this measure for each of the remaining 7 Elastic Sections.

**In-Bag Stack Height Test**

The in-bag stack height of a package of absorbent articles is determined as follows:

Equipment

A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within $\pm$ 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 $\pm$ 1 gram-force (8.34 N) on the absorbent article package, which may be achieved by placing a suitable weight on the center of the non-package-contacting top surface of the horizontal sliding plate so that the total mass of the sliding plate plus added weight is 850 $\pm$ 1grams.

Test Procedure for In-bag Stack Height

Absorbent article packages are equilibrated at 23 $\pm$ 2 °C and 50 $\pm$ 5 % relative humidity prior to measurement.

The horizontal sliding plate is raised and an absorbent article package is placed centrally under the horizontal sliding plate in such a way that the absorbent articles within the package are in a horizontal orientation (see Fig. 6). Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within $\pm$ 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) $\times$ 10 is calculated and reported to within $\pm$ 0.5 mm.

**Test equipment/environment**

A suitable tensile tester such as an MTS Alliance with MTS Testworks version 4.0 or equivalent instrument is used. The tester is equipped with flat clamps that are capable of holding at least the entire transverse length of the side seam should be used. The instrument is calibrated according to the manufacturer's specification. Testing is performed at 23°C $\pm$ 2°C and 50% $\pm$ 2% relative humidity.

**Sample prep**

The side seams of the product are broken to separate the front belt from the back belt. The respective force zones (as described in the Detailed Description of the Invention) are cut away from these belts. Each separated section of the front and back belt will be referred to as a "test sample" herein. All material layers, including the chassis components, should be kept with the test sample. All cut lines are straight, parallel to the transverse direction of the absorbent article. Each test sample needs to have at least one elastomeric material. The widths (a dimension in the longitudinal direction of the absorbent article) of the respective zones are measured.

The length of the test sample is determined. The length measures in the transverse direction of the absorbent article a distance from one end to the other end of a test sample in a fully stretched condition. The fully stretched condition is the condition where the test sample is stretched by the force of 0.1 N/mm multiplied by the width of the test sample. If one or both ends of a test sample are not parallel to the longitudinal direction, the shortest length within the test sample is considered as the length of the test sample.

An adjusted test sample length is defined such that the length of a test sample minus the combined length of any material in the upper and lower clamps. Thus, if a test sample is mounted in the clamp so that 10 mm at each end is held in the clamps, then the adjusted belt length is the measured belt length minus 20 mm.

The test samples are kept unstretched at least for 10 min before the test.

**Test**

For each test sample, the initial gauge length of the tensile tester is set to allow the test sample to be mounted in a relaxed state. The load cell is zeroed to offset the sample weight.

The test sample is stretched in the transverse direction of the absorbent article at a rate of 254 mm/min, and a load (N) is measured within 5 sec after the test sample reaches at 65% of the adjusted test sample length. The transverse force is calculated for each of the force zones according to an equation:

$$\text{A transverse force (N/mm) of a test sample} = \text{Measured value (N) / width of the force zone (mm)}$$

The dimensions and values disclosed herein are not to be understood as being strictly limited to the exact numeral values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

**Claims**

**1.** An absorbent article comprising a belt, wherein the belt comprises a front belt in a front waist region and a back belt in a back waist region and has a ring-like configuration by permanently or refastenably connecting the front belt and the back belt at seams;
the front belt comprising a front inner belt nonwoven and a front outer belt nonwoven; and
the back belt comprising a back inner belt nonwoven and a back outer belt nonwoven;
and further comprising a central panel comprising an absorbent core disposed in a crotch region, the central panel also comprising a topsheet and a backsheet;
wherein the central panel overlaps a portion of the front and back belts;
wherein the front belt comprises a first plurality of elastics in a first section, the first section extending longitudinally from a front end edge of the article to a midpoint of the front waist region, and the front belt comprising a second plurality of elastics in the second section, the second section extending longitudinally from the midpoint of the first waist region to a distal end edge of the front waist region;
wherein the back belt comprising a third plurality of elastics in a third section, the third section extending longitudinally from a distal end edge of the back waist region to a midpoint of the back waist region; and the back belt comprises a fourth plurality of elastics in the fourth section, the fourth section extending longitudinally from the midpoint of the back waist region to a distal end edge of the rear waist region, and

wherein the first plurality of elastics run continuously and transversely from a first side edge of the front waist region to a second side of the front waist region, wherein the first plurality of elastics have a first average spacing between individual elastics of the first plurality of elastics;

wherein the second plurality of elastics run continuously and transversely from the first side edge of the front waist region to the second side of the front waist region, wherein the second plurality of elastics have a second average spacing between individual elastics of the second plurality of elastics;

wherein the third plurality of elastics run continuously and transversely from a first side edge of the back waist region to a second side of the back waist region, wherein the third plurality of elastics have a third average spacing between individual elastics of the third plurality of elastics;

wherein the fourth plurality of elastics run continuously and transversely from the first side edge of the back waist region to the second side of the back waist region, wherein the fourth plurality of elastics have a fourth average spacing between individual elastics of the fourth plurality of elastics;

wherein the second and third plurality of elastics overlap the absorbent core;

a fifth plurality of elastics in the crotch region, wherein the fifth plurality of elastics extend continuously and transversely across the core and have a fifth average spacing between individual elastics of the fifth plurality of elastics;

a sixth plurality of elastics in the crotch region, wherein the sixth plurality of elastics extend continuously and transversely across the core and have a sixth average spacing between individual elastics of the sixth plurality of elastics;

wherein the fifth plurality of elastics is disposed adjacent to the second plurality of elastics and wherein the fifth average spacing of the fifth plurality of elastics is larger than the second average spacing of the second plurality of elastics;

wherein the sixth plurality of elastics is disposed adjacent to the third plurality of elastics and wherein the sixth average spacing of the sixth plurality of elastics is larger than the third average spacing of the third plurality of elastics; and

a mega-gap free from transversely extending elastics and disposed between the fifth and sixth pluralities of elastics, the mega-gap longitudinally extending from about 20 mm to about 50 mm, wherein the mega-gap overlaps a transverse axis (Ti).

2. The absorbent article of claim 1, wherein the front inner belt nonwoven and the back inner belt nonwoven are discrete from each other and do not overlap.

3. The absorbent article according to any of the preceding claims, wherein the front outer belt nonwoven and the back outer belt nonwoven are discrete from each other and do not overlap.

4. The absorbent article of claims 1-2, wherein the front outer belt nonwoven and the back outer belt nonwoven formed from the same continuous nonwoven layer.

5. The absorbent article according to any of the preceding claims, wherein a majority of a surface area of the mega-gap is disposed between the transverse centerline and the fifth plurality of elastics.

6. The absorbent article according to claims 1-4, wherein a majority of a surface area of the mega-gap is disposed between the transverse centerline and the sixth plurality of elastics.

7. The absorbent article according to any of the preceding claims, wherein the fifth plurality of elastics comprises first and second most distal elastics, wherein a gap between the two most distal elastics of the fifth plurality of elastics is from about 8-12 mm.

8. The absorbent article according to any of the preceding claims, wherein the sixth plurality of elastics comprises first and second most distal elastics, wherein a gap between the two most distal elastics of the sixth plurality of elastics is from about 8-12 mm.

9. The absorbent article according to any of the preceding claims, wherein the fifth plurality of elastics comprises second and third most distal elastics, wherein a gap between the second and third most proximal elastics of the fifth plurality of elastics is from about 5-8 mm.

10. The absorbent article according to any of the preceding claims, wherein the sixth plurality of elastics comprises second and third most distal elastics, wherein a gap between the second and third most proximal elastics of the sixth plurality of elastics is from about 5-8 mm.

**11.** The absorbent article according to any of the preceding claims, wherein the fifth average spacing of the fifth plurality of elastics is larger than the first average spacing of the first plurality of elastics.

**12.** The absorbent article according to any of the preceding claims, wherein the sixth average spacing of the sixth plurality of elastics is larger than the third average spacing of the third plurality of elastics.

**13.** The absorbent article according to any of the preceding claims, wherein the sixth average spacing of the sixth plurality of elastics is larger than the fifth average spacing of the fifth plurality of elastics.

**14.** The absorbent article according to any of claims 1-12, wherein the fifth average spacing of the fifth plurality of elastics is larger than the sixth average spacing of the sixth plurality of elastics.

**15.** The absorbent article according to any of the preceding claims, wherein the gaps between the individual elastics of the fifth plurality of elastics get larger along the longitudinal axis in a direction from the distal end edge of the front waist region toward the transverse axis.


**Patentansprüche**

**1.** Absorptionsartikel, umfassend ein Band, wobei das Band ein vorderes Band in einem vorderen Taillenbereich und ein hinteres Band in einem hinteren Taillenbereich umfasst und eine ringartige Konfiguration aufweist, indem das vordere Band und das hintere Band an Nähten permanent oder wiederverschließbar verbunden sind;
wobei das vordere Band ein vorderes inneres Bandvlies und ein vorderes äußeres Bandvlies umfasst; und
wobei das hintere Band ein hinteres inneres Bandvlies und ein hinteres äußeres Bandvlies umfasst;
und ferner umfassend ein Mittelfeld umfassend einen Absorptionskern in einem Schrittbereich, wobei das Mittelfeld auch eine Oberschicht und eine Unterschicht umfasst;
wobei das Mittelfeld einen Abschnitt des vorderen und des hinteren Bands überlappt;
wobei das vordere Band eine erste Vielzahl von Gummibändern in einem ersten Abschnitt umfasst, wobei sich der erste Abschnitt in Längsrichtung von einem vorderen Endrand des Artikels zu einem Mittelpunkt des vorderen Taillenbereichs erstreckt und das vordere Band eine zweite Vielzahl von Gummibändern im zweiten Abschnitt umfasst, wobei sich der zweite Abschnitt in Längsrichtung von dem Mittelpunkt des ersten Taillenbereichs zu einem distalen Endrand des vorderen Taillenbereichs erstreckt;
wobei das hintere Band eine dritte Vielzahl von Gummibändern in einem dritten Abschnitt umfasst, wobei sich der dritte Abschnitt in Längsrichtung von einem distalen Endrand des hinteren Taillenbereichs zu einem Mittelpunkt des hinteren Taillenbereichs erstreckt; und das hintere Band eine vierte Vielzahl von Gummibändern im vierten Abschnitt umfasst, wobei sich der vierte Abschnitt in Längsrichtung von dem Mittelpunkt des hinteren Taillenbereichs zu einem distalen Endrand des hinteren Taillenbereichs erstreckt, und
wobei die erste Vielzahl von Gummibändern ununterbrochen und quer von einem ersten Seitenrand des vorderen Taillenbereichs zu einer zweiten Seite des vorderen Taillenbereichs verläuft, wobei die erste Vielzahl von Gummibändern einen ersten mittleren Abstand zwischen einzelnen Gummibändern der ersten Vielzahl von Gummibändern aufweist;
wobei die zweite Vielzahl von Gummibändern ununterbrochen und quer vom ersten Seitenrand des vorderen Taillenbereichs zur zweiten Seite des vorderen Taillenbereichs verläuft, wobei die zweite Vielzahl von Gummibändern einen zweiten mittleren Abstand zwischen einzelnen Gummibändern der zweiten Vielzahl von Gummibändern aufweist;
wobei die dritte Vielzahl von Gummibändern ununterbrochen und quer von einem ersten Seitenrand des hinteren Taillenbereichs zu einer zweiten Seite des hinteren Taillenbereichs verläuft, wobei die dritte Vielzahl von Gummibändern einen dritten mittleren Abstand zwischen einzelnen Gummibändern der dritten Vielzahl von Gummibändern aufweist;
wobei die vierte Vielzahl von Gummibändern ununterbrochen und quer vom ersten Seitenrand des hinteren Taillenbereichs zur zweiten Seite des hinteren Taillenbereichs verläuft, wobei die vierte Vielzahl von Gummibändern einen vierten mittleren Abstand zwischen einzelnen Gummibändern der vierten Vielzahl von Gummibändern aufweist;
wobei die zweite und dritte Vielzahl von Gummibändern den Absorptionskern überlappen;
eine fünfte Vielzahl von Gummibändern im Schrittbereich, wobei sich die fünfte Vielzahl von Gummibändern ununterbrochen und quer über den Kern erstreckt und einen fünften mittleren Abstand zwischen einzelnen Gummibändern der fünften Vielzahl von Gummibändern aufweist;
eine sechste Vielzahl von Gummibändern im Schrittbereich, wobei sich die sechste Vielzahl von Gummibändern ununterbrochen und quer über den Kern erstreckt und einen sechsten mittleren Abstand zwischen einzelnen Gummibändern der sechsten Vielzahl von Gummibändern aufweist;

wobei die fünfte Vielzahl von Gummibändern benachbart zu der zweiten Vielzahl von Gummibändern angeordnet ist und wobei der fünfte mittlere Abstand der fünften Vielzahl von Gummibändern größer als der zweite mittlere Abstand der zweiten Vielzahl von Gummibändern ist;

wobei die sechste Vielzahl von Gummibändern benachbart zu der dritten Vielzahl von Gummibändern angeordnet ist und wobei der sechste mittlere Abstand der sechsten Vielzahl von Gummibändern größer als der dritte mittlere Abstand der dritten Vielzahl von Gummibändern ist; und

einen Mega-Spalt, der frei von quer verlaufenden Gummibändern und zwischen der fünften und sechsten Vielzahl von Gummibändern angeordnet ist, wobei sich der Mega-Spalt in Längsrichtung von etwa 20 mm bis etwa 50 mm erstreckt, wobei der Mega-Spalt eine Querachse (Ti) überlappt.

2. Absorptionsartikel nach Anspruch 1, wobei das vordere innere Bandvlies und das hintere innere Bandvlies voneinander getrennt sind und einander nicht überlappen.

3. Absorptionsartikel nach einem der vorstehenden Ansprüche, wobei das vordere äußere Bandvlies und das hintere äußere Bandvlies voneinander getrennt sind und einander nicht überlappen.

4. Absorptionsartikel nach einem der Ansprüche 1 bis 2, wobei das vordere äußere Bandvlies und das hintere äußere Bandvlies aus der gleichen ununterbrochenen Vliesschicht gebildet sind.

5. Absorptionsartikel nach einem der vorstehenden Ansprüche, wobei ein Großteil eines Oberflächenbereichs des Mega-Spalts zwischen der Querachse und der fünften Vielzahl von Gummibändern angeordnet ist.

6. Absorptionsartikel nach den Ansprüchen 1 bis 4, wobei ein Großteil eines Oberflächenbereichs des Mega-Spalts zwischen der Querachse und der sechsten Vielzahl von Gummibändern angeordnet ist.

7. Absorptionsartikel nach einem der vorstehenden Ansprüche, wobei die fünfte Vielzahl von Gummibändern ein am weitesten und ein am zweitweitesten distal liegendes Gummiband umfasst, wobei ein Spalt zwischen den beiden am weitesten distal liegenden Gummibändern der fünften Vielzahl von Gummibändern etwa 8 bis 12 mm beträgt.

8. Absorptionsartikel nach einem der vorstehenden Ansprüche, wobei die sechste Vielzahl von Gummibändern ein am weitesten und ein am zweitweitesten distal liegendes Gummiband umfasst, wobei ein Spalt zwischen den beiden am weitesten distal liegenden Gummibändern der sechsten Vielzahl von Gummibändern etwa 8 bis 12 mm beträgt.

9. Absorptionsartikel nach einem der vorstehenden Ansprüche, wobei die fünfte Vielzahl von Gummibändern ein am zweitweitesten und ein am drittweitesten distal liegendes Gummiband umfasst, wobei ein Spalt zwischen dem am zweitweitesten und dem am drittweitesten distal liegenden Gummiband der fünften Vielzahl von Gummibändern etwa 5 bis 8 mm beträgt.

10. Absorptionsartikel nach einem der vorstehenden Ansprüche, wobei die sechste Vielzahl von Gummibändern ein am zweitweitesten und ein am drittweitesten distal liegendes Gummiband umfasst, wobei ein Spalt zwischen dem am zweitweitesten und dem am drittweitesten Gummiband der sechsten Vielzahl von Gummibändern etwa 5 bis 8 mm beträgt.

11. Absorptionsartikel nach einem der vorstehenden Ansprüche, wobei der fünfte mittlere Abstand der fünften Vielzahl von Gummibändern größer als der erste mittlere Abstand der ersten Vielzahl von Gummibändern ist.

12. Absorptionsartikel nach einem der vorstehenden Ansprüche, wobei der sechste mittlere Abstand der sechsten Vielzahl von Gummibändern größer als der dritte mittlere Abstand der dritten Vielzahl von Gummibändern ist.

13. Absorptionsartikel nach einem der vorstehenden Ansprüche, wobei der sechste mittlere Abstand der sechsten Vielzahl von Gummibändern größer als der fünfte mittlere Abstand der fünften Vielzahl von Gummibändern ist.

14. Absorptionsartikel nach einem der Ansprüche 1 bis 12, wobei der fünfte mittlere Abstand der fünften Vielzahl von Gummibändern größer als der sechste mittlere Abstand der sechsten Vielzahl von Gummibändern ist.

15. Absorptionsartikel nach einem der vorstehenden Ansprüche, wobei die Spalte zwischen den einzelnen Gummibändern der fünften Vielzahl von Gummibändern entlang der Längsachse in einer Richtung von dem distalen Endrand des vorderen Taillenbereich in Richtung der Querachse größer werden.

**Revendications**

1. Article absorbant comprenant une ceinture, dans lequel la ceinture comprend une ceinture avant dans une région avant de taille et une ceinture arrière dans une région arrière de taille et a une configuration en forme d'anneau en reliant de manière permanente ou repositionnable la ceinture avant et la ceinture arrière au niveau de coutures ;

la ceinture avant comprenant un non-tissé intérieur avant de ceinture et un non-tissé extérieur avant de ceinture ; et la ceinture arrière comprenant un non-tissé intérieur arrière de ceinture et un non-tissé extérieur arrière de ceinture ; et comprenant en outre un panneau central comprenant une âme absorbante disposée dans une région d'entrejambe, le panneau central comprenant également une feuille de dessus et une feuille de fond ;

dans lequel le panneau central chevauche une partie des ceintures avant et arrière ;

dans lequel la ceinture avant comprend une première pluralité d'élastiques dans une première section, la première section s'étendant longitudinalement depuis un bord d'extrémité avant de l'article jusqu'à un point central de la région avant de taille, et la ceinture avant comprenant une deuxième pluralité d'élastiques dans la deuxième section, la deuxième section s'étendant longitudinalement depuis le point central de la première région de taille jusqu'à un bord d'extrémité distal de la région avant de taille ;

dans lequel la ceinture arrière comprenant une troisième pluralité d'élastiques dans une troisième section, la troisième section s'étendant longitudinalement depuis un bord d'extrémité distal de la région arrière de taille jusqu'à un point central de la région arrière de taille ; et la ceinture arrière comprend une quatrième pluralité d'élastiques dans la quatrième section, la quatrième section s'étendant longitudinalement depuis le point central de la région arrière de taille jusqu'à un bord d'extrémité distal de la région arrière de la taille, et

dans lequel la première pluralité d'élastiques s'étend de manière continue et transversale depuis un premier bord latéral de la région avant de taille jusqu'à un deuxième côté de la région avant de taille, dans lequel la première pluralité d'élastiques ont un premier espacement moyen entre des élastiques individuels de la première pluralité d'élastiques ;

dans lequel la deuxième pluralité d'élastiques s'étend de manière continue et transversale depuis le premier bord latéral de la région avant de taille jusqu'au deuxième côté de la région avant de taille, dans lequel la deuxième pluralité d'élastiques ont un deuxième espacement moyen entre des élastiques individuels de la deuxième pluralité d'élastiques ;

dans lequel la troisième pluralité d'élastiques s'étendent de manière continue et transversale depuis un premier bord latéral de la région arrière de taille jusqu'à un deuxième côté de la région arrière de taille, dans lequel la troisième pluralité d'élastiques ont un troisième espacement moyen entre des élastiques individuels de la troisième pluralité d'élastiques ;

dans lequel la quatrième pluralité d'élastiques s'étendent de manière continue et transversale depuis le premier bord latéral de la région arrière de taille jusqu'au deuxième côté de la région arrière de taille, dans lequel la quatrième pluralité d'élastiques ont un quatrième espacement moyen entre des élastiques individuels de la quatrième pluralité d'élastiques ;

dans lequel les deuxième et troisième pluralité d'élastiques chevauchent l'âme absorbante ;

une cinquième pluralité d'élastiques dans la région d'entrejambe, dans lequel la cinquième pluralité d'élastiques s'étendent de manière continue et transversale à travers l'âme et ont un cinquième espacement moyen entre des élastiques individuels de la cinquième pluralité d'élastiques ;

une sixième pluralité d'élastiques dans la région d'entrejambe, dans lequel la sixième pluralité d'élastiques s'étendent de manière continue et transversale à travers l'âme et ont un sixième espacement moyen entre des élastiques individuels de la sixième pluralité d'élastiques ;

dans lequel la cinquième pluralité d'élastiques est disposée de manière adjacente à la deuxième pluralité d'élastiques et dans lequel le cinquième espacement moyen de la cinquième pluralité d'élastiques est supérieur au deuxième espacement moyen de la deuxième pluralité d'élastiques ;

dans lequel la sixième pluralité d'élastiques est disposée de manière adjacente à la troisième pluralité d'élastiques et dans lequel le sixième espacement moyen de la sixième pluralité d'élastiques est supérieur au troisième espacement moyen de la troisième pluralité d'élastiques ; et

un méga-écart exempt d'élastiques s'étendant transversalement et disposé entre les cinquième et sixième pluralités d'élastiques, le méga-écart s'étendant longitudinalement sur environ 20 mm à environ 50 mm, dans lequel le méga-écart chevauche un axe transversal (Ti).

2. Article absorbant selon la revendication 1, dans lequel le non-tissé intérieur avant de ceinture et le non-tissé intérieur arrière de ceinture sont discrets l'un de l'autre et ne se chevauchent pas.

3. Article absorbant selon l'une quelconque des revendications précédentes, dans lequel le non-tissé extérieur avant de ceinture et le non-tissé extérieur arrière de ceinture sont discrets l'un de l'autre et ne se chevauchent pas.

4. Article absorbant selon les revendications 1 à 2, dans lequel le non-tissé extérieur avant de ceinture et le non-tissé extérieur arrière de ceinture sont formés à partir de la même couche continue de non-tissé.

5. Article absorbant selon l'une quelconque des revendications précédentes, dans lequel une majorité d'une superficie du méga-écart est disposée entre la ligne centrale transversale et la cinquième pluralité d'élastiques.

6. Article absorbant selon les revendications 1 à 4, dans lequel une majorité d'une superficie du méga-écart est disposée entre la ligne centrale transversale et la sixième pluralité d'élastiques.

7. Article absorbant selon l'une quelconque des revendications précédentes, dans lequel la cinquième pluralité d'élastiques comprend des premier et deuxième élastiques les plus distaux, dans lequel un espace entre les deux élastiques les plus distaux de la cinquième pluralité d'élastiques est d'environ 8 à 12 mm.

8. Article absorbant selon l'une quelconque des revendications précédentes, dans lequel la sixième pluralité d'élastiques comprend des premier et deuxième élastiques les plus distaux, dans lequel un espace entre les deux élastiques les plus distaux de la sixième pluralité d'élastiques est d'environ 8 à 12 mm.

9. Article absorbant selon l'une quelconque des revendications précédentes, dans lequel la cinquième pluralité d'élastiques comprend des deuxième et troisième élastiques les plus distaux, dans lequel un espace entre les deuxième et troisième élastiques les plus proximaux de la cinquième pluralité d'élastiques est d'environ 5 à 8 mm.

10. Article absorbant selon l'une quelconque des revendications précédentes, dans lequel la sixième pluralité d'élastiques comprend des deuxième et troisième élastiques les plus distaux, dans lequel un espace entre les deuxième et troisième élastiques les plus proximaux de la sixième pluralité d'élastiques est d'environ 5 à 8 mm.

11. Article absorbant selon l'une quelconque des revendications précédentes, dans lequel le cinquième espacement moyen de la cinquième pluralité d'élastiques est supérieur au premier espacement moyen de la première pluralité d'élastiques.

12. Article absorbant selon l'une quelconque des revendications précédentes, dans lequel le sixième espacement moyen de la sixième pluralité d'élastiques est supérieur au troisième espacement moyen de la troisième pluralité d'élastiques.

13. Article absorbant selon l'une quelconque des revendications précédentes, dans lequel le sixième espacement moyen de la sixième pluralité d'élastiques est supérieur au cinquième espacement moyen de la cinquième pluralité d'élastiques.

14. Article absorbant selon l'une quelconque des revendications 1 à 12, dans lequel le cinquième espacement moyen de la cinquième pluralité d'élastiques est supérieur au sixième espacement moyen de la sixième pluralité d'élastiques.

15. Article absorbant selon l'une quelconque des revendications précédentes, dans lequel les espaces entre les élastiques individuels de la cinquième pluralité d'élastiques sont supérieurs le long de l'axe longitudinal dans une direction allant du bord d'extrémité distal de la région avant de taille vers l'axe transversal.

Fig. 1

Fig. 2

Fig. 3A

Fig. 3B

Fig. 3C

Fig. 3D

FIG. 4A

FIG. 4B

Fig. 5A

Fig. 5B

Fig. 6

# EP 3 451 991 B1

## REFERENCES CITED IN THE DESCRIPTION

### Patent documents cited in the description

- US 20160100999 A
- US 14878156 B
- US 20L60270976 A
- US 62136003 B
- US 20080132872 A
- US 11999229 B
- US 20170290713 A
- US 62319463 B
- US 5518801 A, Chappell
- US 5366782 A, Curro
- US 20160270976 A
- US 8939957 B, Raycheck
- US 6590136 B, Young
- US 5599335 A, Goldman
- US 5562646 A, Goldman
- US 5669894 A, Goldman
- US 6790798 B, Suzuki
- US 521587 A, Busam
- US 20040158212 A, Ponomarenko
- US 8536401 B, Ecker
- US 6989006 B, LaVon
- US 7381202 B, LaVon
- US 7175613 B, Sugiyama
- US 7824386 B, LaVon
- US 7766887 B, Burns
- US 6989005 B, LaVon
- US 6649810 B, Minato
- US 7073373 B, La Fortune
- US 6383960 B, Everett
- US 5810796 A, Kimura
- US 6689934 B, Dodge
- US 8568566 B, Jackels
- US 2012316046 A, Jackels
- US 2014027066 A
- US 8979815 B, Roe
- US 9216118 B, Roe
- US 9216116 B, Roe
- US 2012316526 A, Rosati
- US 2012316527 A, Rosati
- US 2012316528 A, Kreuzer
- US 2012316529 A, Kreuzer
- US 2012316523 A, Hippe
- US 2014163501 A, Ehrnsperger
- US 2014163502 A, Arizti
- US 2014163503 A, Arizti
- EP 2532328 A
- EP 2532329 A
- EP 2717823 A
- EP 2717820 A
- EP 2717821 A
- EP 2717822 A
- EP 2532332 A
- EP 2740449 A
- EP 2740452 A
- US 7901393 B, Matsuda